# EUROPÄISCHE PATENTSCHRIFT

(11) **EP 2 166 940 B1**
(45) Veröffentlichungstag und Bekanntmachung des Hinweises auf die Patenterteilung: **01.02.2012**
(21) Anmeldenummer: 08760523.4
(22) Anmeldetag: 04.06.2008
(51) Int. Cl.: A61B 5/0492, A61B 5/12, A61N 1/05, A61N 1/36, H04R 25/00

(54) **ELEKTRODENANORDNUNG UND MESSVORRICHTUNG ZUR MESSUNG DER ELEKTRISCHEN AKTIVITÄT IN EINEM ELEKTRISCH AKTIVEN GEWEBE**
ELECTRODE ARRANGEMENT AND MEASURING DEVICE FOR MEASURING THE ELECTRICAL ACTIVITY IN AN ELECTRICALLY ACTIVE TISSUE
ENSEMBLE D'ELECTRODES ET DISPOSITIF DE MESURE DESTINE A MESURER L'ACTIVITE ELECTRIQUE DANS UN TISSU ELECTRIQUEMENT ACTIF

(30) Priorität: 05.06.2007 DE 102007026645
(43) Veröffentlichungstag der Anmeldung: 31.03.2010
(73) Patentinhaber: Med-El Elektromedizinische Geräte GmbH, 6020 Innsbruck (AT)
(72) Erfinder: BEHREND, Detlef, 18119 Rostock-Warnemünde (DE); SCHMITZ, Klaus-Peter, 18119 Rostock-Warnemünde (DE); PAU, Hans Wilhelm, 18055 Rostock (DE); STERNBERG, Katrin, 18055 Rostock (DE); SCHMIDT, Wolfram, 18109 Rostock (DE)
(74) Vertreter: Lucke, Andreas
(86) Internationale Anmeldenummer: PCT/EP2008/056944
(87) Internationale Veröffentlichungsnummer: WO 2008/148822

(56) Entgegenhaltungen:
- DE-A1-102007 008 154
- US-A- 4 590 946
- US-A- 6 157 861
- US-A1- 2005 216 073
- US-B1- 6 205 360
- US-B1- 6 208 882
- CLEMENT R S ET AL: "Characteristics of stapedius muscle electromyograms elicited by cochlear implant stimulation in the rat" PROCEEDINGS OF THE 26TH ANNUAL INTERNATIONAL CONFERENCE OF THE IEEE ENGINEERING IN MEDICINE AND BIOLOGY SOCIETY. IEEE ENGINEERING IN MEDICINE AND BIOLOGY SOCIETY. CONFERENCE 2004, Bd. 6, 2004, Seiten 4221-4224, XP002495109 San Francisco, CA, USA ISSN: 1557-170X

## Beschreibung

Die vorliegende Erfindung betrifft eine Elektrodenanordnung sowie eine Messvorrichtung zur Messung des Aktionsstroms und/oder des Aktionspotenzials eines elektrisch aktiven Gewebes, insbesondere betrifft die vorliegende Erfindung eine bipolare Stapediusmuskel-Elektrodenanordnung zur Messung des bei einer Kontraktion des Stapediusmuskels erzeugten Aktionspotentials.

Das menschliche Ohr lässt sich in die Bereiche Außenohr (Ohrmuschel), Mittelohr und Innenohr unterteilen. Das Mittelohr besteht aus dem Trommelfell und den Gehörknöchelchen Hammer, Amboss und Steigbügel. Das Trommelfell wird über das Außenohr einlaufenden Schallwellen in Schwingung versetzt. Diese Schwingungen können nun über Hammer, Amboss und Steigbügel an das ovale Fenster des Innenohrs übertragen werden, wodurch wiederum in der Flüssigkeit der Gehörschnecke Schallschwingungen erzeugt werden. Durch die Bewegung der Flüssigkeit werden in die Gehörschnecke ragende Haarzellen gebogen und lösen dabei Nervenimpulse aus. Im Mittelohr findet eine mechanische Impedanzwandlung statt, die eine optimale Übertragung des Schallsignals vom Außenohr zum Innenohr ermöglicht.

Darüber hinaus befinden sich im Mittelohr der Trommelfellspannmuskel und der so genannte Stapediusmuskel. Der Trommelfellspannmuskel ist am Hammer angelenkt, wobei der Stapediusmuskel über eine Sehne mit dem Steigbügel verbunden ist. Im Falle eines zu hohen Schalldrucks, welcher das Innenohr schädigen könnte, kontrahieren beide Muskeln reflexartig, so dass die mechanische Ankopplung des Trommelfells an das Innenohr (und somit auch die Kraftübertragung) verringert wird. Hierdurch ist ein Schutz des Innenohrs vor zu hohen Schalldrücken möglich. Die in Folge hoher Schalldrücke ausgelöste Anspannung des Stapediusmuskels wird auch als Stapediusreflex bezeichnet. Aus der Diagnose des Stapediusreflexes lassen sich medizinisch relevante Informationen über die Funktionsfähigkeit des Ohres gewinnen. Weiterhin ist die Messung des Stapediusreflexes zur Einstellung bzw. Kalibrierung so genannter Cochlearimplantate nützlich, da aus dem gemessenen Stapediusreflex auf die von einem Patienten wahrgenommene Schallenergie geschlossen werden kann.

Es ist bekannt, zur Messung des Stapediusreflexes Elektroden einzusetzen, die mit dem Stapediusmuskel in Kontakt gebracht werden und die bei einer Kontraktion des Stapediusmuskels erzeugten Aktionsströme bzw. Aktionspotentiale an eine Messvorrichtung weiterleiten. Dabei ist eine zuverlässige, minimal-invasive Kontaktierung des Stapediusmuskels schwierig, da der Stapediusmuskel innerhalb einer in einem Knochen vorhandenen Mulde angeordnet ist und lediglich die mit dem Steigbügel verbundene Sehne des Stapediusmuskeis sowie dessen oberer Teil vom Inneren des Mittelohrs aus zugänglich sind.

Aus US 6,208,882 sind unterschiedliche Stapediusmuskelektroden bekannt. Diese erreichen jedoch nur eine unzureichende Kontaktierung des Stapediusmuskelgewebes (insbesondere bei Muskelkontraktion) und sind weiterhin stark traumatisierend.

Weitere verwandte Elektroden sind aus der US 2005/216073 A1, der US 6 205 360 B1 und der US 4 590 946 A bekannt.

Es ist daher Aufgabe der vorliegenden Erfindung, eine einfache und preiswert herstellbare Elektrode zur Messung von Aktionsströmen und/oder Aktionspotenzialen in elektrisch aktiven Geweben (vorzugsweise dem Stapediusmuskelgewebe) anzugeben, die einerseits eine sichere, jedoch reversible Fixierung der Elektrode im Muskelgewebe gewährleistet und andererseits das Muskelgewebe möglichst wenig traumatisiert.

Diese Aufgaben werden erfindungsgemäß durch die Merkmale des Patentanspruchs 1 gelöst. Zweckmäßige Ausgestaltungen der Erfindung sind in den Unteransprüchen enthalten.

Erfindungsgemäß ist die Elektrodenanordnung mindestens zweiteilig ausgebildet und weist eine erste Elektrode und ein Fixierungselement auf, wobei die erste Elektrode mit einer ersten, länglichen elektrischen Leitung verbunden ist, und wobei die erste Elektrode weiterhin aus einem langgestreckten Grundkörper mit einem ersten Ende und einem zweiten Ende besteht, wobei die erste elektrische Leitung mit dem Grundkörper im Bereich dessen zweiten Endes verbunden ist, und wobei Mittel zur Fixierung des Fixierungselements an der ersten Elektrode vorgesehen sind. Die Idee der Erfindung besteht darin, die Fixierung der (ersten) Elektrode im (Stapedius-) Muskelgewebe durch ein separat einzubringendes Fixierungselement zu bewirken, wobei das Fixierungselement den Grundkörper der (ersten) Elektrode vorzugsweise durch dessen Mantelfläche durchdringt und ihn damit (beispielsweise auf der Sehne des Stapediusmuskels) fixiert.

In einer besonders bevorzugten Ausführungsvariante sind der Grundkörper der (ersten) Elektrode und das Fixierungselement derart ausgebildet, dass das Fixierungselement nahezu senkrecht (bevorzugt mit einem Winkel zwischen 45 und 90° zur Längsachse des Grundkörpers) in den Grundkörper der (ersten) Elektrode einrastbar, einsteckbar oder einschraubbar ist. Dabei soll die Verbindung zwischen Fixierungselement und Grundkörper reversibel, also wieder lösbar sein. Der Vorteil der Erfindung gegenüber herkömmlichen Elektroden besteht darin, dass die (erste) Elektrode aufgrund der seitlichen Fixierung über keine weiteren (traumatisierenden) Haltemechanismen verfügen muss und daher sehr leichtgängig und somit für das Gewebe minimal-invasiv ausgebildet sein kann.

Es ist ferner bevorzugt, dass eine bipolare Messung des Aktionspotentials des Gewebes (Stapediusmuskelgewebe) erfolgt, wobei das Fixierungselement als (vorzugsweise langgestreckte) zweite Elektrode ausgebildet ist. Dadurch kann das Aktionspotential mit hoher örtlicher Auslösung bestimmt werden, nämlich zwischen zwei Messpunkten der beiden Elektroden. Diese beiden Elektroden sind dazu vorzugsweise gegeneinander (in dem Bereich, in dem sie sich direkt berühren) isoliert. Vorzugsweise besteht wenigstens eine der beiden Elektroden aus einem leitenden Kern und weist eine isolierende Ummantelung auf, wobei der leitende Kern jeweils im Bereich des gewünschten Messpunktes freigelegt ist. Diese freigelegten Bereiche befinden sich außerhalb des Gebietes, in dem sich die beiden Elektroden zur Fixierung direkt kontaktieren.

Vorzugsweise ist der Grundkörper im Wesentlichen halbhohlzylinderförmig (oder halbzylinderförmig) ausgebildet. Unter einem im Wesentlichen halbhohlzylinderförmigen Grundkörper wird, ein (nicht nur an den Kopfenden, sondern auch) entlang seiner Längsachse offener, hohlzylinderförmiger Grundkörper verstanden, wobei die Mantelfläche im Querschnitt kreisbogenförmig ausgebildet ist, und wobei der Mittelpunktswinkel des Kreisbogens zwischen 120° und 240°, bevorzugter zwischen 165° und 195° und besonders bevorzugt 180° beträgt. Der zum Kreisbogen zugehörige Radius beträgt vorzugsweise zwischen 0.25 und 1.5 mm. Die Wandstärke der Mantelfläche beträgt vorzugsweise zwischen 50 und 500 µm.

Weiterhin ist es bevorzugt, dass der Grundkörper aus einem elektrisch leitfähigen starren Material ausgebildet ist (bevorzugte Biegesteifigkeit 200-600 N mm², noch bevorzugter 400-500 N mm² und besonders bevorzugt 450 N mm²). In einer bevorzugten Ausführungsvariante wird der elektrisch leitfähige Grundkörper durch eine Isolierung (mit Ausnahme des freigelegten Bereichs - vorzugsweise im Bereich des spitz zulaufenden ersten Endes) ummantelt.

Vorzugsweise weist der Grundkörper auf seiner Mantelfläche oder auf seiner äußeren Oberfläche eine elektrisch isolierte Durchgangsöffnung auf. Der Querschnitt der Durchgangsöffnung korrespondiert mit dem Querschnitt der zweiten Elektrode derart, dass die zweite Elektrode in die Durchgangsöffnung einführbar, einrastbar, einsteckbar oder einschraubbar ist. Durch die korrespondierenden Abmessungen von Durchgangsöffnung der ersten Elektrode und dem Querschnitt der zweiten Elektrode wird es erfindungsgemäß ermöglicht, dass die erste Elektrode durch einführen, einrasten, einstecken oder einschrauben der zweiten Elektrode in die Durchgangsöffnung temporär oder dauerhaft und sicher fixiert werden kann. Insbesondere lässt sich die Fixierung durch rückwärtiges Herausziehen der zweiten Elektrode in einfacher Weise lösen, so dass beide Elektroden nach der Messung des Aktionspotentials/- stroms einfach herausgezogen werden können. Da erfindungsgemäß keine weiteren Fixierungselemente an den Elektroden vorgesehen sind, kann eine Messung des Gewebe(Muskel)-Reflexes minimal-invasiv erfolgen.

Vorzugsweise beträgt die laterale Ausdehnung der Durchgangsöffnung zwischen 101% und 110% der lateralen Ausdehnung der zweiten Elektrode. Dadurch wird ein ausreichendes Spiel zum Einführen/Herausziehen der zweiten Elektrode gewährleistet, jedoch eine sichere Fixierung der Elektroden sichergestellt. Vorzugsweise weist die Durchgangsöffnung eine elektrische Isolationsschicht auf ihrer Innenfläche auf, damit ein Kurzschluss im Kontaktbereich der Elektroden ausgeschlossen ist. Vorzugsweise besteht die Isolationsschicht aus einer Isolationskeramik oder aus Saphir und weist eine Dicke zwischen 10 und 30 µm auf. Alternativ ist es auch möglich, dass die zweite Elektrode eine elektrische Isolierung in dem Bereich aufweist, in dem sie die Durchgangsöffnung des Grundkörpers kontaktiert. Diese elektrische Isolierung besteht vorzugsweise aus Silikon oder Polyuhrethan.

Vorzugsweise weist die zweite Elektrode einen Durchmesser von 0.2 mm bis 2 mm auf. Vorzugsweise weist der Grundkörper eine Länge von 0,8 mm bis 1,4 mm auf. Vorzugsweise weist die zweite Elektrode eine Länge von 1.5 mm bis 3.0 mm auf. Die bevorzugten Dimensionen sind insbesondere geeignet, dass die erste Elektrode gewebeschonend innerhalb des zwischen Sehne/Stapediusmuskel und Knochen befindlichen Kanals geführt werden kann. Insbesondere kann der halbhohlzylinderförmige Grundkörper der ersten Elektrode die Sehne des Stapediusmuskels (halb) umschließen und dadurch (wie auf einer Führung) sicher zum Muskel vorgeschoben werden. Die zweite, zur Fixierung dienende Elektrode wird vorzugsweise senkrecht dazu in das Gewebe (Sehne) eingeführt.

Der Grundkörper weist im Bereich seines ersten Endes vorzugsweise einen Anschliff auf, der einen Winkel zwischen 30° und 60° zur Längsachse des Grundkörpers besitzt. Damit wird eine gewebeschonende Einführung der ersten Elektrode ermöglicht.

Sofern das Fixierungselement aus einem elektrisch isolierenden Material besteht (also keine zweite Elektrode vorhanden ist) kann die erste Elektrode als monopolare Elektrode ausgebildet sein. Dann ist die erste elektrische Leitung vorzugsweise form- und/oder kraftschlüssig mit einem Spannungsmessgerät oder einem Strommessgerät verbunden. Vorzugsweise ist das Spannungsmessgerät dann zur Messung des an der ersten Elektrode anliegenden elektrischen Potentials in Bezug zu einem Referenzpotential oder Nullpotential ausgelegt.

In einer besonders bevorzugten Ausführungsvariante ist es vorgesehen, dass die erste Elektrode und die zweite Elektrode als bipolare Messanordnung ausgebildet sind. Dann sind sowohl die erste elektrische Leitung als auch die zweite Elektrode mit einem Spannungsmessgerät/Strommessgerät verbunden und das Spannungsmessgerät ist zur Messung des zwischen erster Elektrode und zweiter Elektrode anliegenden elektrischen Potentials ausgelegt. Vorzugsweise ist das Spannungsmessgerät zur Messung des zwischen erster Elektrode und zweiter Elektrode anliegenden elektrischen Potentials im Bereich von +100 mV bis -100 mV (bevorzugt +40 mV bis -90 mV) ausgelegt.

Neben der ersten Elektrode kann auch die zweite Elektrode mit einer zweiten elektrischen (Zu-)Leitung verbunden sein, wobei die Zuleitungen mit dem (Spannungs- oder Strom-)Messgerät verbunden sind. Die Elektroden müssen eine gewisse Eigensteifigkeit im distalen Bereich besitzen, um mit bekannten OP-Instrumenten gehalten und geführt zu werden. Die Zuleitungen sind vorzugsweise biegeschlaff ausgeführt.

Die erste Elektrode kann vollständig elektrisch leitfähig sein, solange die Zuführung gegenüber dem umliegenden Gewebe elektrisch isoliert ist. Natürlich muss für den bipolaren Fall auch eine Isolation gegenüber der zweiten Elektrode vorliegen. Dazu kann die zweite Elektrode teilweise isoliert sein oder die Durchgangsöffnung (Bohrung) der ersten Elektrode.

Der Elektrodenanordnung der Erfindung findet Verwendung in einem Verfahren zur Bestimmung des Aktionsstroms und/oder des Aktionspotentials eines menschlichen, elektrisch aktiven Gewebes offenbart, bei dem eine erste (mit den o.g. Merkmalen versehene) Elektrode aus einer ersten Richtung in das aktive Gewebes und ein Fixierungselement (vorzugsweise eine zweite, mit den o.g. Merkmalen versehene Elektrode) separat aus einer zweiten Richtung in das aktive Gewebes eingeführt, wobei das Fixierungselement (vorzugsweise die zweite Elektrode) in die erste Elektrode eingesteckt, eingerastet oder eingeschraubt wird. Vorzugsweise wird das Fixierungselement (vorzugsweise die zweite Elektrode) unter einem Winkel von 70°- 90° zur Längsachse der ersten Elektrode eingesteckt, eingerastet oder eingeschraubt.

Im Falle der Messung des Aktionspotentials des Stapediusmuskels wird zunächst die erste Elektrode direkt in das Muskelgewebe eingeführt. Im Falle des Stapediusmuskels erfolgt dies entlang der Sehne des Muskels, der damit als Führung dient. Die zweite Elektrode dient der Fixierung und wird demnach separat und senkrecht zur Längsachse der ersten Elektrode und der Sehne eingeführt. Der Muskel ist im OP-Gebiet zugänglich. Um die Durchgangsöffnung zu treffen, kann die Sehne als Orientierung dienen. Als zweite Variante kann die Fixierung nicht im Muskel, sondern außerhalb auf der Sehne erfolgen. In diesem Fall ist die Durchgangsöffnung sichtbar und leicht zu treffen. Die zweite Elektrode kann trotzdem als Bezugselektrode für eine bipolare Potenzialableitung verwendet werden. Sollten für die Applikation weitere Gewebe penetriert werden, ist das für die Funktion unerheblich. Alle Elektroden und Zuleitungen sind vorzugsweise gegenüber anderen Geweben oder Körperflüssigkeiten elektrisch isoliert.

Die Erfindung soll nachstehend anhand von zumindest teilweise in den Figuren dargestellten Ausführungsbeispielen näher erläutert werden.

Es zeigen:
- Fig. 1: die erste Elektrode einer erfindungsgemäßen zweiteiligen Elektrodenanordnung in schematischer, geschnittener Darstellung,
- Fig. 2: eine erfindungsgemäße Elektrodenanordnung in schematischer, geschnittener Darstellung, bei der die zweite Elektrode zur Fixierung in die erste Elektrode eingesteckt ist,
- Fig.3: die erfindungsgemäße Elektrodenanordnung gemäß Fig. 2 in schematischer Schnittdarstellung senkrecht zur Längsachse der ersten Elektrode,
- Fig. 4a: die an einer Stapediusmuskelsehne befestigte, erfindungsgemäße Elektrodenanordnung in perspektivischer Darstellung,
- Fig. 4b: die an einer Stapediusmuskelsehne befestigte, erfindungsgemäße Elektrodenanordnung in geschnittener Darstellung,
- Fig. 5: die erfindungsgemäße Elektrodenanordnung gemäß Fig. 2 in Draufsicht,
- Fig. 6: eine erfindungsgemäße Messvorrichtung in schematischer Darstellung, wobei die erste in den Stapediusmuskel und die zweite Elektrode in die Sehne eingeführt werden (vor und nach der Fixierung/Messung), und
- Fig. 7: eine erfindungsgemäße Messvorrichtung in schematischer Darstellung, wobei erste und zweite Elektrode im Stapediusmuskel fixiert sind (während der Messung).

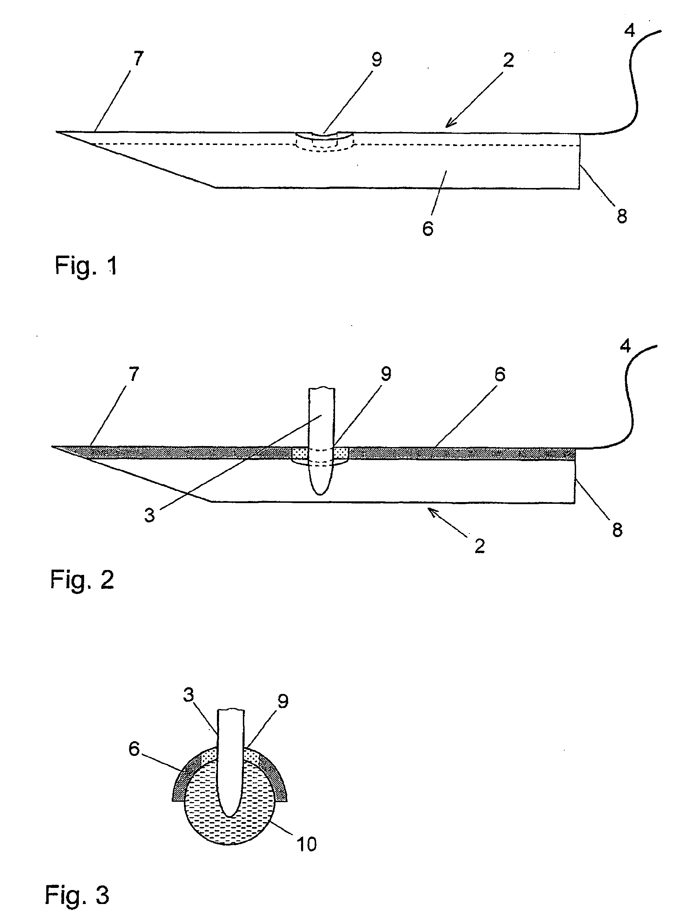

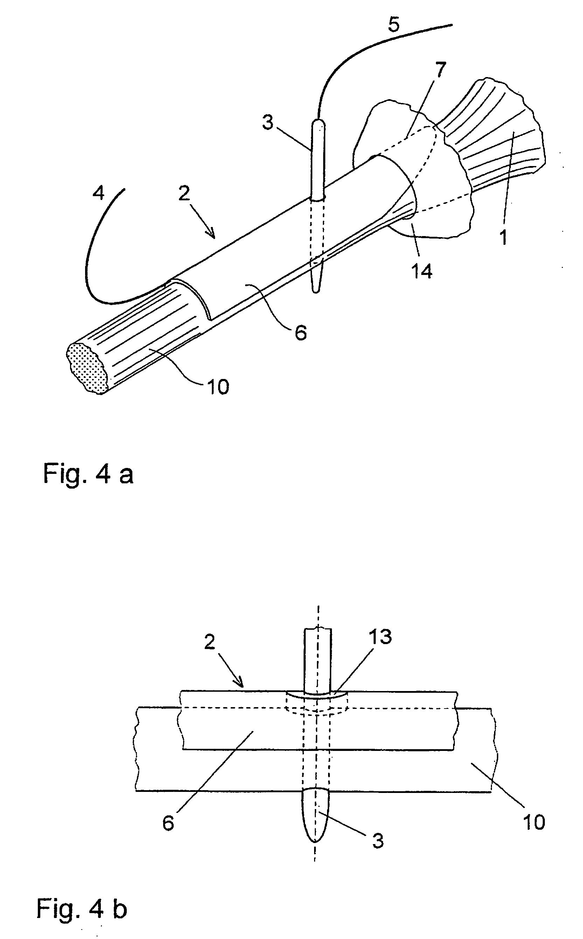

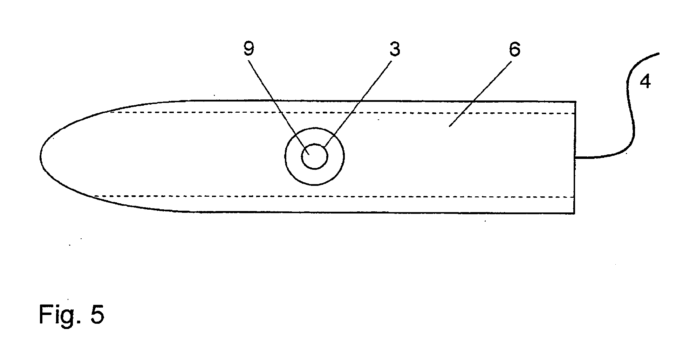

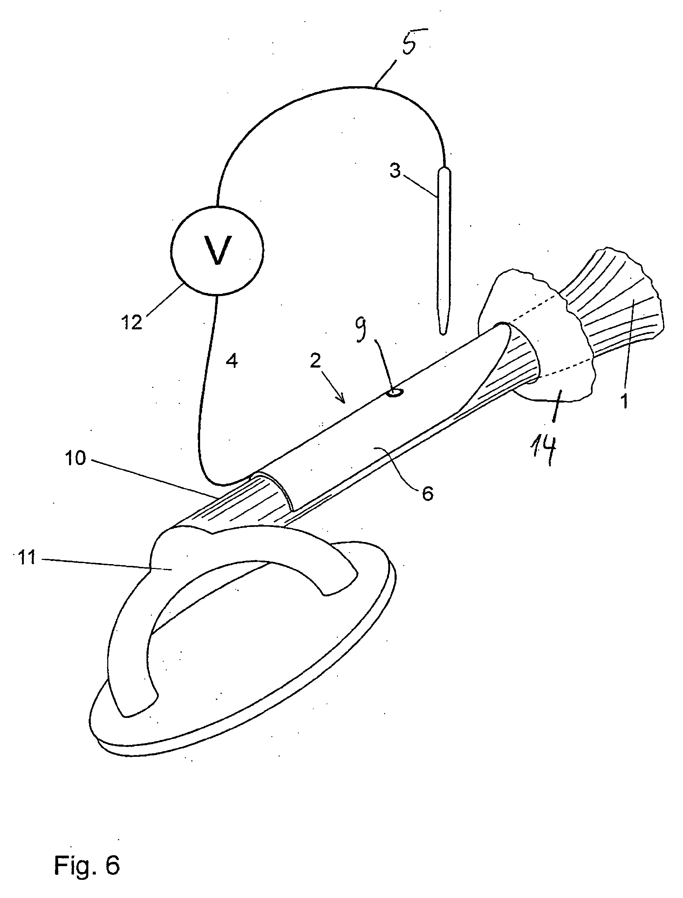

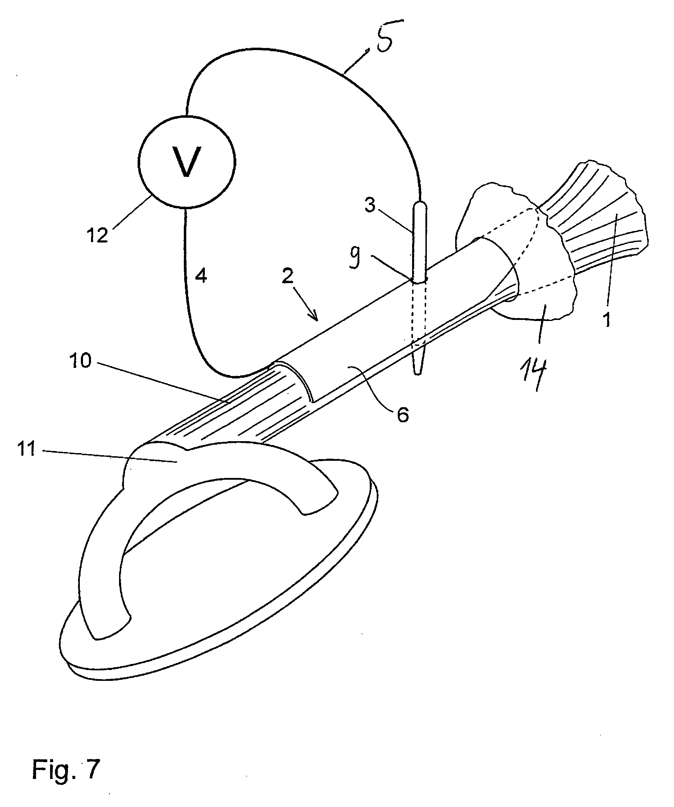

Fig. 1 zeigt eine (erste) Elektrode 2 der erfindungsgemäßen Elektrodenanordnung in schematischer, geschnittener Darstellung. Die erste Elektrode 2 besteht erfindungsgemäß aus einem langgestreckten Grundkörper 6, der in einer besonders bevorzugten Ausführungsvariante halbhohlzylinderförmig (entlang seiner Längsachse offener Hohlzylinder mit einem Mittelpunktswinkel von circa 180°) aus einem leitenden Material ausgebildet ist. Ferner ist es vorgesehen, dass eine elektrische Zuleitung 4 im Bereich des zweiten Endes 8 mit dem Grundkörper 6 verbunden ist. Die elektrische Zuleitung 4 ist vorzugsweise elektrisch isoliert. Im Bereich des ersten Endes 7 weist der hohlzylinderförmige Grundkörper 6 vorzugsweise einen Anschliff im Winkelbereich von 30° bis 60° auf. Aufgrund seiner länglichen Form ist eine Durchführung des Grundkörpers 6 lediglich entlang seiner Längsachse durch ein Gewebe 1 möglich. Um eine sichere Fixierung des Grundkörpers 6 in einem elektrisch aktiven Gewebe 1 zu gewährleisten, ist es erfindungsgemäß vorgesehen, den Grundkörper 6 mittels eines separaten, in Bezug auf die Längsachse des Grundkörpers 6 abgewinkelt einzuführenden (angeordneten) Fixierungselements 3 zu fixieren. In einer besonders bevorzugten Ausführungsvariante (Fig. 2 bis 7) wird die Fixierung durch eine zweite Elektrode 3 realisiert, welche in einer besonders bevorzugten Ausführungsvariante in eine Durchgangsöffnung 9 des Grundkörpers 6 eingesteckt wird. Dadurch kann eine bipolare Messanordnung geschaffen werden, wobei die erste Elektrode 2 über die elektrische Zuleitung 4 und die zweite Elektrode 3 über die elektrische Zuleitung 5 jeweils mit einem Spannungsmessgerät 12 (Fig. 6 und 7) verbunden sind. Aufgrund der vorhandenen Beweglichkeit des Grundkörpers 6 entlang seiner Längsachse innerhalb des Muskelgewebes 1 wird eine sichere Fixierung mittels der zweiten Elektrode 3 durch Einstecken dieser in die Durchgangsöffnung 9 unter einem zur Längsachse des Grundkörpers 6 endlichen Winkels (vorzugsweise 60°-90°) erreicht, da die eingesteckte zweite Elektrode 3 die Bewegung des Grundkörpers 6 entlang seiner Längsachse blockiert. Besonders bevorzugt ist es, die zweite Elektrode 3 senkrecht in die Durchgangsöffnung 9 des Grundkörpers 6 einzustecken. Alternativ ist es auch möglich, zwischen den Elektroden 2, 3 eine Schraub- oder Rastverbindung herzustellen. Zur Messung des Aktionspotentials innerhalb des Gewebes ist es notwendig, dass die Elektroden 2, 3 in demjenigen Bereich, in dem sie sich direkt kontaktieren, voneinander elektrisch isoliert sind. So ist es beispielsweise möglich, dass der Grundkörper 6 elektrisch leitend ausgebildet und lediglich im Bereich 13 seiner Durchgangsöffnung 9 elektrisch isoliert ist. Befindet sich nur die Spitze (erstes Ende 7) des Grundkörpers 6 im Muskelgewebe 1 (Fig. 4b und 7), so ist es jedoch vorteilhaft, auch den restlichen Grundkörper 6 (außer im Bereich des ersten Endes 7) zu isolieren. Die elektrische Zuleitung 4 (und/oder die elektrische Zuleitung 5) muss ebenfalls gegenüber umliegendem Gewebe stets elektrisch isoliert sein. Alternativ zur Isolierung des Grundkörpers 6 im Bereich der Durchgangsöffnung 9 ist es möglich, die Elektrode 3 in dem Bereich, in dem sie in die Durchgangsöffnung 9 eingesteckt wird, zu isolieren. Die zweite Elektrode 3 bzw. deren elektrische Zuführung 5 sowie die erste Elektrode 2 bzw. deren elektrische Zuführung 4 (Fig. 4a) müssen jedenfalls in einem zum Messgerät 12 verlaufenden Bereich elektrisch isoliert sein, um eine punktuelle Messung des Aktionspotentials im Stapediusmuskel 1 - Fig. 7 - realisieren zu können.

Fig. 6 und 7 zeigen eine mögliche Verwendung der erfindungsgemäßen Elektrodenanordnung zur Messung des Aktionspotentials des Stapediusmuskels 1. Dazu ist es vorgesehen, den Grundkörper 6 der ersten Elektrode 2 mittels entsprechender OP-Instrumente entlang der Sehne 10, welche den Stapediusmuskel 1 mit dem Steigbügel 11 verbindet, in den Stapediusmuskel 1 zu bewegen (siehe 6 und 7 und auch Fig. 4a). Dabei kann der Elektrodengrundkörper 6 in den (zwischen der Sehne 10 und dem den Stapediusmuskel 1 umgebenden Knochen 14 vorhandenen) Kanal geschoben werden (Fig. 7). Die zweite Elektrode 3 wird erfindungsgemäß separat und (nahezu) senkrecht zur Vorschubrichtung des Elektrodengrundkörpers 6 in den Stapediusmuskel 1 (alternativ durch die Durchgangsöffnung 9 in die Sehne 10) geschoben. Beide Elektroden 2, 3 werden so weit eingeschoben, bis das erste Ende 7 des Grundkörpers 6 den Stapediusmuskel 1 mindestens partiell kontaktiert und bis die zweite Elektrode 3 in die Durchgangsöffnung 9 des Elektrodengrundkörpers 6 eingreift (Fig. 7) und dadurch eine Bewegung des Elektrodenkörpers 6 entlang seiner Längsachse blockiert. Nun ist eine sichere Bestimmung des Aktionspotentials über das angeschlossene Messgerät 12 möglich. Jedoch muss die erste Elektrode 2 nicht vollständig in den Stapediusmuskel 1 eingeschoben werden; alternativ ist es auch möglich, den Elektrodengrundkörper 6 lediglich partiell in den Stapediusmuskel 1 einzuführen (Fig. 4a und 7). In diesem Fall soll der Elektrodengrundkörper 6 elektrisch isoliert (außer im Bereich des vorderen Endes 7) sein. Nun kann die zweite Elektrode 3 außerhalb des Stapediusmuskels 1 (im Bereich der Sehne 10) in die Durchgangsöffnung 9 eingeschoben werden und den Elektrodengrundkörper 6 zur Messung des Aktionspotentials fixieren. Vorzugsweise ist der innere Bereich 13 der Durchgangsöffnung 9 mit einer Isolierung versehen, damit ein Kurzschluss zwischen erster Elektrode 2 und zweiter Elektrode 3 vermieden werden kann (Fig. 4b).

### Bezugszeichenliste

- 1: elektrisch aktives Gewebe/Muskel
- 2: erste Elektrode
- 3: Fixierungselement/zweite Elektrode
- 4: erste elektrische Leitung
- 6: Grundkörper
- 7: erstes Ende des Grundkörpers
- 8: zweites Ende des Grundkörpers
- 9: Durchgangsöffnung
- 10: Sehne
- 11: Steigbügel-Knöchel
- 12: Spannungsmessgerät/ Strommessgerät
- 13: Isolierung
- 14: Knochen

## Patentansprüche

1. Elektrodenanordnung zur Messung des Aktionsstroms und/oder des Aktionspotentials eines elektrisch aktiven Gewebes (1), aufweisend: eine erste Elektrode (2) und ein Fixierungselement (3), wobei die erste Elektrode (2) mit einer ersten, länglichen elektrischen Leitung (4) verbunden ist, und wobei die erste Elektrode (2) aus einem langgestreckten Grundkörper (6) mit einem ersten Ende (7) und einem zweiten Ende (8) besteht, wobei die erste elektrische Leitung (4) mit dem Grundkörper (6) im Bereich dessen zweiten Endes (8) verbunden ist, und wobei Mittel zur Fixierung des Fixierungselements (3) an der ersten Elektrode (2) vorgesehen sind,
**dadurch gekennzeichnet, dass** der Grundkörper (6) auf einer Mantelfläche oder auf einer äußeren Oberfläche eine Durchgangsöffnung (9) aufweist, wobei der Querschnitt der Durchgangsöffnung (9) mit dem Querschnitt des Fixierungselementes (3) derart korrespondiert, dass das Fixierungselement (3) in die Durchgangsöffnung (9) einführbar oder einsteckbar ist.

2. Elektrodenanordnung nach Anspruch 1,
**dadurch gekennzeichnet, dass**
das Fixierungselement (3) als langgestreckte, zweite Elektrode (3) ausgebildet ist, wobei
die zweite Elektrode (3) vorzugsweise mit einer zweiten, länglichen elektrischen Leitung verbunden ist.

3. Elektrodenanordnung nach einem der vorhergehenden Ansprüche,
**dadurch gekennzeichnet, dass**
der Grundkörper (6) die Form eines entlang seiner Längsachse offenen, Hohlzylinders aufweist, wobei die Mantelfläche im Querschnitt kreisbogenförmig ausgebildet ist, und wobei der Mittelpunktswinkel des Kreisbogens zwischen 165° und 195° beträgt und/oder der Grundkörper (6) aus einem starren Material ausgebildet ist.

4. Elektrodenanordnung nach Anspruch 2,
**dadurch gekennzeichnet, dass**
die laterale Ausdehnung der Durchgangsöffnung (9) zwischen 100 % und 200 % vorzugsweise zwischen 100 % und 150 % und besonders vorzugsweise zwischen 101 % und 130 % der lateralen Ausdehnung der zweiten Elektrode (3) aufweist, und/oder die Durchgangsöffnung (9) eine elektrische Isolationsschicht auf ihrer Innenfläche aufweist, wobei
die Isolationsschicht aus Isolationskeramik, insbesondere A₂O₃, TiO₂ oder einer Glaskeramik oder Saphir besteht und/oder eine Dicke zwischen 10 und 30 µm aufweist.

5. Elektrodenanordnung nach Anspruch 4,
**dadurch gekennzeichnet, dass**
die zweite Elektrode (3) eine elektrische Isolierung gegenüber der Durchgangsöffnung (9) des Grundkörpers (6) aufweist, wobei
die elektrische Isolierung vorzugsweise aus Silikon- oder Polyurethanelastomeren besteht.

6. Elektrodenanordnung nach einem der vorhergehenden Ansprüche,
**dadurch gekennzeichnet, dass**
der Grundkörper (6) einen Durchmesser von 0,5 mm bis 3,0 mm aufweist, und/oder der Grundkörper (6) eine Länge von 0,8 mm bis 1,4 mm aufweist.

7. Elektrodenanordnung nach einem der Ansprüche 2 bis 6,
**dadurch gekennzeichnet, dass**
die zweite Elektrode (3) einen Durchmesser von 0,2 mm bis 2 mm aufweist, und/oder der Grundkörper (6) eine Länge von 0,8 mm bis 1,4 mm aufweist.

8. Elektrodenanordnung nach einem der vorhergehenden Ansprüche,
**dadurch gekennzeichnet, dass**
die erste Elektrode (2) aus CrCoMo, Pt, PtIr, Ti, TiA14V6 besteht und/oder die zweite Elektrode (3) aus dem gleichen Material wie die erste Elektrode (2) besteht, wobei erste elektrische Leitung (4) vorzugsweise aus dem gleichen Material wie die erste Elektrode (2) besteht und/oder die zweite elektrische Leitung (5) aus dem gleichen Material wie die erste elektrische Leitung (4) besteht.

9. Elektrodenanordnung nach einem der Ansprüche 2 bis 7,
**dadurch gekennzeichnet, dass**
die erste elektrische Leitung (4) eine Länge von 10 mm bis 100 cm und die zweite elektrische Leitung (5) eine Länge von 10 mm bis 100 cm aufweist, und/oder die erste elektrische Leitung (4) mittels einer Löt-, Klemm- oder Schweißverbindung mit der ersten Elektrode (2) verbunden ist, und/oder
der Grundkörper (6) im Bereich seines ersten Endes (7) einen Anschliff aufweist, der einen Winkel zwischen 10° und 80° zur Längsachse des Grundkörpers (6) aufweist, und/oder
der Grundkörper (6) im Bereich seines ersten Endes (7) einen Anschliff aufweist, der einen Winkel zwischen 30° und 60° zur Längsachse des Grundkörpers (6) aufweist.

10. Elektrodenanordnung nach einem der vorhergehenden Ansprüche,
**dadurch gekennzeichnet, dass**
die erste Elektrode (2) als monopolare Elektrode ausgebildet ist und das Fixierungselement (3) aus einem elektrisch isolierenden Material besteht, wobei
die erste elektrische Leitung (4) vorzugsweise form- und/oder kraftschlüssig mit einem Spannungsmessgerät (12) oder einem Strommessgerät verbunden ist, und/oder
die erste elektrische Leitung (4) mittels eines Verbindungssteckers als lösbare Klemm-, Steck- oder Schraubverbindung mit einem Spannungsmessgerät (12) oder einem Strommessgerät verbunden ist.

11. Elektrodenanordnung nach einem der Ansprüche 2 bis 9,
**dadurch gekennzeichnet, dass**
die erste Elektrode (2) und die zweite Elektrode (3) als bipolare Messanordnung ausgebildet sind, wobei
die erste elektrische Leitung (4) und die zweite Elektrode (3) vorzugsweise mit einem Spannungsmessgerät (12) oder einem Strommessgerät verbunden sind, wobei vorzugsweise
das Spannungsmessgerät (12) zur Messung des an der ersten Elektrode (2) anliegenden elektrischen Potentials in Relation zu einem Referenzpotential ausgelegt ist und/oder
das Spannungsmessgerät (12) zur Messung des an der Elektrode anliegenden elektrischen Potentials in Bezug zum Nullpotential (Ground) ausgelegt ist, oder
das Spannungsmessgerät (12) zur Messung des zwischen erster Elektrode (2) und zweiter Elektrode (3) anliegenden elektrischen Potentials ausgelegt ist.

12. Elektrodenanordnung nach einem der Ansprüche 2 bis 11,
**dadurch gekennzeichnet, dass**
der längliche Elektrodengrundkörper (1) und/oder die erste elektrische Leitung (4) einen elektrisch leitenden Kern sowie einen elektrisch isolierenden Mantel aufweist, und/oder
die zweite Elektrode (3) einen elektrisch leitenden Kern sowie einen elektrisch isolierenden Mantel aufweist, wobei
die Isolierung vorzugsweise eine Wandstärke zwischen 2 und 30 µm aufweist und/oder die Isolierung aus einem biokompatiblen Elastomer besteht.

13. Elektrodenanordnung nach Anspruch 12,
**dadurch gekennzeichnet, dass**
der elektrisch leitende Kern des Elektrodengrundkörpers (1) lediglich im Bereich der zweiten Elektrode freigelegt ist, wobei der elektrisch leitende Kern des Elektrodengrundkörpers (1) den elektrisch leitenden Kern der zweiten Elektrode (3) nicht direkt kontaktiert, oder
der elektrisch leitende Kern der zweiten Elektrode (3) lediglich im Bereich des Elektrodengrundkörpers (1) freigelegt ist, wobei der elektrisch leitende Kern des Elektrodengrundkörpers (1) den elektrisch leitenden Kern der zweiten Elektrode (3) nicht direkt kontaktiert.

14. Elektrodenanordnung nach Anspruch 13,
**dadurch gekennzeichnet, dass**
der freigelegte Bereich eine Fläche von 0,02 bis 15 mm² aufweist.

## Claims

1. An electrode arrangement for measuring the action current and/or the action potential of an electrically active tissue (1), comprising: a first electrode (2) and a fixing element (3), the first electrode (2) being connected to a first, oblong electrical line (4), and the first electrode (2) consisting of an elongate main body (6) having a first end (7) and a second end (8), the first electrical line (4) being connected to the main body (6) in the region of its second end (8), and means being provided for fixing the fixing element (3) on the first electrode (2), **characterised in that** the main body (6) comprises a through-opening (9) on a circumferential surface or on an outer surface, the cross-section of the through-opening (9) corresponding to the cross-section of the fixing element (3) in such a way that the fixing element (3) can be introduced or inserted into the through-opening (9).

2. The electrode arrangement according to claim 1, **characterised in that** the fixing element (3) is formed as an elongate second electrode (3), the second electrode (3) preferably being connected to a second, oblong electrical line.

3. The electrode arrangement according to any one of the preceding claims, **characterised in that** the main body (6) has the form of a hollow cylinder which is open along its longitudinal axis, the circumferential surface being provided in the form of a circular arc in cross-section, and the angle at centre of the circular arc being between 165° and 195° and/or the main body (6) being formed from a rigid material.

4. The electrode arrangement according to claim 2, **characterised in that** the lateral extension of the through-opening (9) is between 100 % and 200 %, preferably between 100 % and 150 % and more preferably between 101 % and 130 % of the lateral extension of the second electrode (3), and/or the through-opening (9) comprises an electrical insulation layer on its inner surface, the insulation layer consisting of insulation ceramics, in particular A₂O₃, TiO₂, or a glass ceramic or sapphire and/or having a thickness between 10 and 30 µm.

5. The electrode arrangement according to claim 4, **characterised in that** the second electrode (3) has an electrical insulation in relation to the through-opening (9) of the main body (6), the electrical insulation preferably consisting of silicone elastomers or polyurethane elastomers.

6. The electrode arrangement according to one of the preceding claims, **characterised in that** the main body (6) has a diameter of 0.5 mm to 3.0 mm, and/or the main body (6) has a length of 0.8 mm to 1.4 mm.

7. The electrode arrangement according to one of claims 2 to 6, **characterised in that** the second electrode (3) has a diameter of 0.2 mm to 2 mm and/or the main body (6) has a length of 0.8 mm to 1.4 mm.

8. The electrode arrangement according to any one of the preceding claims, **characterised in that** the first electrode (2) consists of CrCoMo, Pt, PtIr, Ti, TiAl4V6, and/or the second electrode (3) consists of the same material as the first electrode (2), the first electrical line (4) preferably consisting of the same material as the first electrode (2) and/or the second electrical line (5) consisting of the same material as the first electrical line (4).

9. The electrode arrangement according to one of claims 2 to 7, **characterised in that** the first electrical line (4) has a length of 10 mm to 100 cm and the second electrical line (5) has a length of 10 mm to 100 cm, and/or the first electrical line (4) is connected to the first electrode (2) by means of a soldered, clamped, or welded connection, and/or the main body (6) has a bevel in the region of its first end (7), which bevel has an angle between 10° and 80° to the longitudinal axis of the main body (6), and/or the main body (6) has a bevel in the region of its first end (7), which bevel has an angle between 30° and 60° to the longitudinal axis of the main body (6).

10. The electrode arrangement according to one of the preceding claims, **characterised in that** the first electrode (2) is formed as a monopolar electrode and the fixing element (3) consists of an electrically insulating material, the first electrical line (4) preferably being connected with a positive and/or non-positive fit to a voltmeter (12) or a current meter, and/or the first electrical line (4) being connected to a voltmeter (12) or current meter by means of a connecting plug as a detachable clamped, plug-in, or screwed connection.

11. The electrode arrangement according to one of claims 2 to 9, **characterised in that** the first electrode (2) and the second electrode (3) are formed as a bipolar measuring arrangement, the first electrical line (4) and the second electrode (3) preferably being connected to a voltmeter (12) or a current meter, the voltmeter (12) preferably being designed to measure the electrical potential applied to the first electrode (2) in relation to a reference potential and/or the voltmeter (12) being designed to measure the electrical potential applied to the electrode in relation to the zero potential (ground), or the voltmeter (12) being designed to measure the electrical potential applied between the first electrode (2) and the second electrode (3).

12. The electrode arrangement according to one of claims 2 to 11, **characterised in that** the oblong electrode main body (1) and/or the first electrical line (4) have an electrically conductive core and an electrically insulating sheath, and/or the second electrode (3) has an electrically conductive core and an electrically insulating sheath, the insulation preferably having a wall thickness between 2 and 30 µm and/or the insulation consisting of a biocompatible elastomer.

13. The electrode arrangement according to claim 12, **characterised in that** the electrically conductive core of the electrode main body (1) is only exposed in the region of the second electrode, the electrically conductive core of the electrode main body (1) not directly contacting the electrically conductive core of the second electrode (3), or the electrically conductive core of the second electrode (3) is only exposed in the region of the electrode main body (1), the electrically conductive core of the electrode main body (1) not directly contacting the electrically conductive core of the second electrode (3).

14. The electrode arrangement according to claim 13, **characterised in that** the exposed region has an area of 0.02 to 15 mm².

## Revendications

1. Ensemble d'électrodes pour la mesure du courant d'action et/ou du potentiel d'action d'un tissu (1) électriquement actif présentant : une première électrode (2) et un élément de fixation (3), la première électrode (2) étant reliée à un premier câble électrique allongé (4), et la première électrode (2) étant constituée d'un corps de base (6) étiré en longueur comportant une première extrémité (7) et une seconde extrémité (8), le premier câble électrique (4) étant relié avec le corps de base (6) dans la partie de la seconde extrémité (8), et des moyens pour fixer les éléments de fixation (3) étant prévus sur la première électrode (2)
**caractérisé en ce que** le corps de base (6) présente un orifice de passage (9) sur une surface d'enveloppe ou sur une surface extérieure, la section transversale de l'orifice de passage (9) correspondant à la section transversale de l'élément de fixation de telle sorte que l'élément de fixation (3) peut être introduit ou enfoncé dans l'orifice de passage (9).

2. Ensemble d'électrodes selon la revendication 1
**caractérisé en ce que**
l'élément de fixation (3) est en forme d'une seconde électrode (3) étirée en longueur,
la seconde électrode (3) étant de préférence reliée avec un second câble électrique allongé.

3. Ensemble d'électrodes selon l'une des revendications précédentes
**caractérisé en ce que**
le corps de base (6) présente la forme d'un cylindre creux ouvert le long de son axe longitudinal, la surface d'enveloppe dans la section transversale étant conçue en forme d'un arc de cercle et l'angle du point central de l'arc de cercle étant entre 165 ° et 195 ° et/ou le corps de base (6) étant constitué d'un matériau rigide.

4. Ensemble d'électrodes selon la revendication 2
**caractérisé en ce que**
l'extension latérale de l'orifice de passage (9) représente entre 100 % et 200 %, de préférence entre 100 % et 150 %, et de manière particulièrement préférée entre 101 % et 130 % de l'extension latérale de la seconde électrode (3), et/ou que l'orifice de passage (9) présente une couche d'isolement électrique sur sa surface interne,
la couche d'isolement étant constituée d'une céramique d'isolement, en particulier d'Al₂O₃, de TiO₂ ou d'une céramique de verre ou de saphir et/ou présentant une épaisseur entre 10 et 30 µm.

5. Ensemble d'électrodes selon la revendication 4
**caractérisé en ce que**
la seconde électrode (3) présente un isolement électrique en face de l'orifice de passage (9) du corps de base (6),
l'isolement électrique étant de préférence constitué par des élastomères de silicones ou de polyuréthane.

6. Ensemble d'électrodes selon l'une des revendications précédentes
**caractérisé en ce que**
le corps de base (6) présente un diamètre entre 0,5 mm et 3,0 mm, et/ou
le corps de base (6) présente une longueur entre 0,8 mm et 1,4 mm.

7. Ensemble d'électrodes selon l'une des revendications de 2 à 6
**caractérisé en ce que**
la seconde électrode (3) présente un diamètre entre 0,2 mm et 2 mm, et/ou
le corps de base (6) présente une longueur entre 0,8 mm et 1,4 mm.

8. Ensemble d'électrodes selon l'une des revendications précédentes
**caractérisé en ce que**
la première électrode (2) est constituée de CrCoMo, Pt, PtIr, Ti, TiAl4V6 et/ou la seconde électrode (3) est constituée du même matériau que la première électrode (2),
une première ligne électrique (4) étant de préférence constituée du même matériau que la première électrode (2) et/ou le second câble électrique (5) étant constitué du même matériau que le premier câble électrique (4).

9. Ensemble d'électrodes selon l'une des revendications de 2 à 7
**caractérisé en ce que**
le premier câble électrique (4) présente une longueur entre 10 mm et 100 cm et le second câble électrique (5) présente une longueur entre 10 mm et 100 cm, et/ou
le premier câble électrique (4) est relié à la première électrode (2) au moyen d'une liaison par brasage, par serrage ou par soudage, et/ou
le corps de base (6) présente un affûtage qui possède un angle entre 10 ° et 80 ° par rapport à l'axe longitudinal du corps de base (6), et/ou
le corps de base (6) présente un affûtage dans la partie de sa première extrémité (7), affûtage qui possède un angle entre 30 ° et 60 ° par rapport à l'axe longitudinal du corps de base (6).

10. Ensemble d'électrodes selon l'une des revendications précédentes
**caractérisé en ce que**
la première électrode (2) est conçue comme une électrode monopolaire et que l'élément de fixation (3) est constitué d'un matériau isolant électrique, le premier câble électrique (4) étant de préférence relié par complémentarité de formes ou par une liaison de force avec un appareil de mesure de la tension (12) ou avec un appareil de mesure du courant, et/ou
le premier câble électrique (4) étant relié avec un appareil de mesure de tension (12) ou avec un appareil de mesure du courant au moyen d'un commutateur de liaison sous la forme d'une liaison par serrage, par introduction ou par vissage, pouvant être désengagé.

11. Ensemble d'électrodes selon l'une des revendications de 2 à 9
**caractérisé en ce que**
la première électrode (2) et la seconde électrode sont disposées sous la forme d'un ensemble de mesure bipolaire,
le premier câble électrique (4) et la seconde électrode (3) étant de préférence reliés avec un appareil de mesure de la tension (12) ou avec un appareil de mesure du courant,
l'appareil de mesure de la tension (12) étant de préférence en place pour la mesure du potentiel électrique appliqué à la première électrode (2) par rapport à un potentiel de référence et/ou
l'appareil de mesure de la tension (12) étant en place pour la mesure du potentiel électrique appliqué à l'électrode par rapport au potentiel zéro (terre), ou
l'appareil de mesure de la tension (12) étant en place pour la mesure du potentiel électrique appliqué entre la première électrode (2) et la seconde électrode (3).

12. Ensemble d'électrodes selon l'une des revendications de 2 à 11,
**caractérisé en ce que**
le corps de base de l'électrode (1) de forme allongée et/ou le premier câble électrique (4) présentent un coeur non conducteur électriquement ainsi qu'une enveloppe isolante électriquement, et/ou
la seconde électrode (3) présente un coeur conducteur électrique ainsi qu'une enveloppe isolante électriquement,
l'isolement présentant de préférence une épaisseur de paroi entre 2 et 30 µm et/ou
l'isolement étant constitué par un élastomère biocompatible.

13. Ensemble d'électrodes selon la revendication 12
**caractérisé en ce que**
le coeur conducteur électrique du corps de base d'électrode (1) se trouve libéré purement et simplement au niveau de la seconde électrode, le coeur conducteur électrique du corps de base d'électrode (1) n'étant pas directement en contact avec le coeur conducteur électrique de la seconde électrode (3), ou
le coeur conducteur électrique de la seconde électrode (3) se trouve purement et simplement libéré dans une zone du corps de base d'électrode (1), le coeur conducteur électrique du corps de base d'électrode (1) n'étant pas en contact direct avec le coeur conducteur électrique de la seconde électrode (3).

14. Ensemble d'électrodes selon la revendication 13
**caractérisé en ce que**
le domaine libéré présente une surface entre 0,02 et 15 mm².
